# FASCICULE DE BREVET EUROPEEN

(11) **EP 3 577 434 B1**
(45) Date de publication et mention de la délivrance du brevet: **03.06.2026**
(21) Numéro de dépôt: 18705106.5
(22) Date de dépôt: 01.02.2018
(51) Int. Cl.: G01N 1/10, G01N 1/40, G01N 33/18

(54) **DISPOSITIF DE FIXATION D'ESPECES CHIMIQUES DANS UN MILIEU AQUATIQUE NATUREL ET PROCEDE DE MISE EN OEUVRE**
VORRICHTUNG ZUR FIXIERUNG CHEMISCHER EINHEITEN IN EINEM NATÜRLICHEN AQUATISCHEN MEDIUM UND IMPLEMENTIERUNGSVERFAHREN
DEVICE FOR FIXING CHEMICAL ENTITIES IN A NATURAL AQUATIC MEDIUM, AND IMPLEMENTATION PROCESS

(30) Priorité: 03.02.2017 FR 1770116
(43) Date de publication de la demande: 11.12.2019
(73) Titulaire: Analytical And Environmental Laboratory / Laboratoire, 98800 Noumea (NC)
(72) Inventeur: FERNANDEZ, Jean-Michel, 98800 Noumea (NC)
(74) Mandataire: Aquinov
(86) Numéro de dépôt international: PCT/EP2018/052547
(87) Numéro de publication internationale: WO 2018/141862

(56) Documents cités:
- CN-U- 202 994 514
- CN-U- 204 630 756
- CN-U- 204 964 242
- CN-Y- 201 222 009
- FR-A1- 2 996 642
- US-A- 4 464 574
- US-A1- 2015 362 409
- GERALDINE S. C. TURNER ET AL: "Evaluation of DGT as a long-term water quality monitoring tool in natural waters; uranium as a case study", ENVIRON. SCI.: PROCESSES IMPACTS, vol. 16, no. 3, 13 January 2014 (2014-01-13), pages 393 - 403, XP055416177, ISSN: 2050-7887, DOI: 10.1039/c3em00574g

## Description

La présente invention concerne un dispositif de fixation d'espèces chimiques dans un milieu naturel aquatique, notamment un milieu marin.

On entend pour la suite de la description comme "espèces chimiques" prises au sens large, des cations métalliques en solution qui est la cible première de la présente demande mais aussi des composés organochlorés.

L'invention couvre aussi le procédé de mise en œuvre du dispositif selon la présente invention. Les milieux naturels aquatiques sont régulièrement affectés par des exploitations humaines qui sont par ailleurs nécessaires pour d'autres activités.

C'est le cas par exemple des activités minières, surtout quand elles sont proches de la mer et que des rejets sont conduits par les écoulements naturels directement vers le milieu marin ou même par des conduites artificielles dédiées qui permettent d'éloigner les rejets plus au large. Afin de déterminer l'impact de telles activités et de prendre les mesures qui s'imposent, si nécessaire, il faut pouvoir disposer de mesures fiables de certaines concentrations en certains éléments minéraux, notamment les concentrations en métaux lourds parmi lesquels le Cadmium Cd, le Cobalt Co, le Chrome Cr, le Cuivre Cu, le Fer Fe, le manganèse Mn, le Nickel Ni, le Plomb Pb et/ou le Zinc Zn.

Ces opérations de fixation doivent être suivies si l'on souhaite assurer un contrôle dans le temps avec une répétitivité, une fiabilité et une reproductibilité satisfaisantes.

En effet, en milieu naturel aquatique, cela se complique du fait des variations de nombreux paramètres qui influencent les mesures et la fixation.

Il existe des moyens techniques pour échantillonner des taux de cations métalliques dans l'eau et notamment il existe des échantillonneurs dits passifs commercialisé sous la dénomination DGT (Diffusive Gradient in Thin film) c'est-à-dire des échantillonneurs comprenant des résines de capture des cations métalliques dans le milieu liquide.

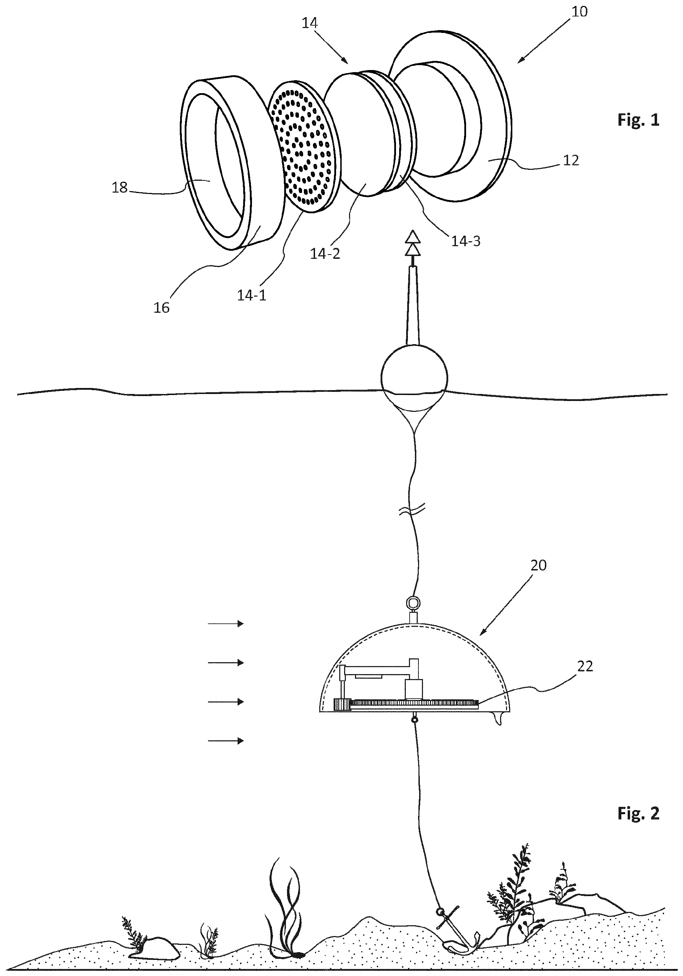

Ces échantillonneurs DGT se présentent sous la forme représentée sur la figure 1, de 4 cm de diamètre pour donner un ordre de grandeur. Ces DGT comprennent un boîtier 10 avec deux parties, une base 12 destinée à recevoir une succession de composants 14 superposés et un couvercle 16 destiné à venir se monter, par coincement mécanique, sur la base 12 de façon étanche, ledit couvercle étant muni d'une fenêtre 18 d'exposition desdits composants.

La succession des trois composants superposés dans chaque échantillonneur comprend, de la fenêtre 18 d'exposition vers la base, trois éléments :
- Une membrane formant filtre 14-1,
- Un gel de diffusion 14-2, et
- Une résine chimique de capture 14-3, apte à capter les espèces chimiques concernées.

La membrane filtre 14-1 a comme fonction de limiter le passage des matières en suspension, MES.

Le gel de diffusion 14-2 permet la diffusion des espèces chimiques vers la résine chimique de capture 14-3.

La résine chimique de capture 14-3 est du type échangeuse d'ions afin de capter de façon irréversible dans le milieu naturel les espèces chimiques pour lesquelles elles sont prévues. De façon connue, on assure ultérieurement la libération des espèces chimiques de ladite résine chimiques de capture.

Ainsi par exemple pour l'application à des cations métalliques, la libération des cations métalliques capturés par la résine s'effectue en laboratoire par échanges en milieu acide fort, les cations métalliques étant remplacés par des ions H⁺. L'analyse se poursuit par spectrométrie à plasma à couplage inductif ou ICP (Inductively Coupled Plasma) pour déterminer les quantités de chacun des cations métalliques.

Ces DGT sont des capteurs qui fonctionnent sans énergie, ils sont passifs, et permettent de concentrer les espèces chimiques dans une solution, de manière intégrée dans le temps.

De tels échantillonneurs sont d'une grande efficacité en théorie mais dans la pratique, on constate que plusieurs paramètres viennent perturber très fortement l'échantillonnage durant l'immersion, les échantillonnages étant alors potentiellement fortement faussés ou à moins insuffisamment précis.

Le premier paramètre est la formation d'un biofilm BF.

En milieu marin, un biofilm se forme très rapidement au bout d'une durée d'exposition de quelques jours mais devient très important au bout d'une dizaine de jours seulement pour donner un ordre d'idées, en fonction des conditions du milieu aquatique naturel concerné.

On constate que le biofilm capte aussi des ions métalliques, et suivant les cas, le biofilm joue le rôle de barrière ou de concentrateur, mais dans tous les cas fausse les résultats.

Cette capture varie au cours du temps et du fait du milieu, on ne peut donc pas appliquer systématiquement un paramètre simple, proportionnel à la durée d'immersion par exemple. En effet, de nombreux paramètres peuvent influencer le développement de ce biofilm comme la température, les courants, l'oxygénation, la richesse de l'eau en matière organique, la salinité et/ou la lumière pour ne citer que ceux-là. Il est donc impossible d'apporter un correctif mathématique sous forme d'un algorithme ou d'un simple coefficient.

Par contre, on peut déterminer les durées d'exposition optimisées, en fonction du lieu, pendant lesquelles la formation d'un biofilm n'influence pas les mesures de façon significative. Un tel constat amène à des durées d'exposition très limitées, ce qui induit une gestion des échantillonneurs DGT complexe, chronophage et coûteuse car il faut immerger les DGT, les retirer, en positionner d'autres, ceci très régulièrement, induisant un grand nombre de sorties en mer des personnels en charge des opérations. Il a été imaginé des supports à échantillonneurs multiples mais ceci ne résout pas le problème, sans compter qu'il faut souvent dupliquer voire analyser en triplicat pour effectuer une moyenne. Dans ce cas, comment disposer au même moment les différents échantillonneurs surtout lorsqu'ils sont éloignés les uns des autres, sauf à recourir à différents plongeurs synchronisés ce qui devient difficilement gérable tant en logistique, qu'en coût financier. Ceci est un autre problème à résoudre.

Il faut aussi rappeler que le milieu marin ne permet pas toujours de sortir en mer dans des conditions satisfaisantes pour gérer de tels échantillonneurs au moment voulu et si le temps est dépassé, comment gérer la reproductibilité, la régularité et la justesse des mesures sachant que le biofilm se développe de façon permanente ?

Surtout qu'un autre problème s'ajoute en parallèle, relatif aux membranes elles-mêmes, si on laisse trop longtemps pour les raisons de mauvaises conditions de récupération au moment déterminé. En effet, les résines de capture exposées au milieu naturel aquatique se saturent au-delà d'une certaine quantité d'espèces chimiques capturées si bien qu'il est alors impossible d'utiliser les données ou du moins ces données ne sont plus significatives dès lors que la membrane est saturée.

L'article "Evaluation of DGT as a long-term water quality monitoring tool in natural waters; uranium as a case study" par Geraldine S.C. Turner et al. décrit l'évaluation d'échantillonneurs DGT comme outil pour surveiller la qualité de l'eau sur de longues périodes.

La présente invention vise un dispositif qui pallie les inconvénients de l'art antérieur en permettant d'utiliser des DGT existants mais qui évite une exposition de ces échantillonneurs à la formation d'un biofilm perturbateur, qui permet un contrôle exact du temps d'exposition pour la fixation des espèces chimiques recherchées, qui évite une surexposition des résines de capture et donc qui évite la saturation.

Le dispositif selon la présente invention est défini dans la revendication indépendante 1. Le procédé selon la présente invention, de mise en œuvre du dispositif selon la présente invention, est défini dans la revendication indépendante 11. Des modes de réalisation préférés sont définis dans les revendications dépendantes.

Le dispositif selon la présente invention peut aussi être utilisé suivant un procédé de séquençage adapté.

Le dispositif autorise une exposition programmée, répétitive, tant pour le départ de l'exposition que pour sa durée ou sa fréquence de répétition contrairement aux échantillonneurs immergés qui sont exposés en permanence et qui travaille en tout ou rien.

Le dispositif évite les manipulations multiples, pour l'immersion comme pour la collecte, collecte qui peut être différée en cas d'intempérie, sans pour autant modifier les données accumulées. De fait les membranes ne subissent pas de saturation liée à une durée d'exposition puisque celle-ci est définie et se trouve déliée du temps d'immersion. Le temps d'exposition est garantie, ceci indépendamment du temps d'immersion.

Le dispositif est autonome en énergie et, sur de plus longues périodes, peut être couplé à une source de production d'énergie électrique.

Le dispositif selon la présente invention permet aussi de collecter d'autres paramètres parallèlement afin de pouvoir corréler les données avec la température par exemple pour corriger finement les données si nécessaire. En effet, la prise de mesures de température en continu par exemple, permet de corriger les séries de données temporelles, si nécessaire.

Les dispositif et procédé selon l'invention sont maintenant décrits en détail selon un mode de réalisation particulier, non limitatif, cette description étant élaborée en regard des dessins annexés, dessins sur lesquels les différentes figures représentent respectivement :
- Figure 1 : une vue éclatée d'un échantillonneur DGT
- Figure 2 : une vue en perspective du dispositif selon la présente invention, installé en milieu naturel, en l'occurrence en mer,
- Figure 3 : une vue mécanique éclatée du dispositif de la figure 2, isolé, hors du milieu naturel,
- Figure 4 : une vue en coupe du dispositif selon la présente invention, en dehors de la fenêtre d'exposition,
- Figure 5 : une vue en coupe du dispositif selon la présente invention, au droit de la fenêtre d'exposition,
- Figures 6A à 6F : des vues de la formation d'un biofilm sur des DGT, en fonction du temps, à 1, 2, 4, 7, 14, 28 jours, et
- Figure 7 : un schéma des perturbations engendrées par le biofilm BF sur la migration des cations métalliques, et
- Figure 8 : une variante d'agencement du dispositif avec une occultation par translation.

Le dispositif 20 selon la présente invention est apte à être immergé en milieu naturel aquatique tel que le milieu marin. Un mouillage comprenant un lien d'amarrage ancré et une bouée par exemple permettent de supporter le dispositif 20 selon la présente invention. Ces moyens ne font pas partie de la présente invention et peuvent être modifiés ou adaptés en fonction des besoins, des contraintes, du lieu etc, il peut même être prévu des moyens fixes. Le but est de positionner ledit dispositif à une profondeur donnée, en un lieu précis et de le maintenir dans cette position, comme montré sur la figure 2.

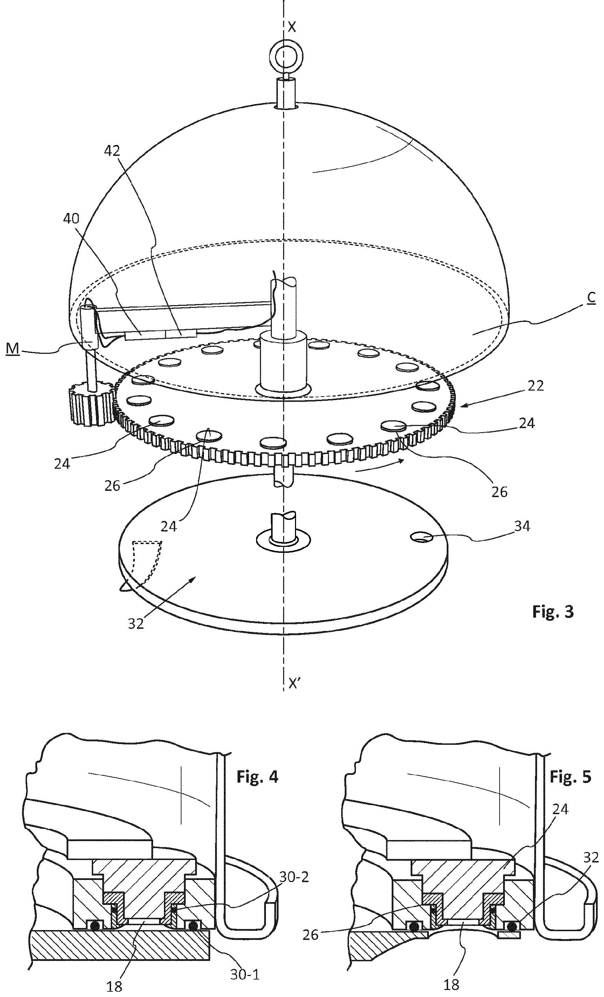

Le dispositif 20 est détaillé sur la figure 3 qui montre une vue en éclaté dudit dispositif.

Le dispositif comprend un plateau 22 support d'échantillonneurs 24 en l'occurrence des échantillonneurs DGT, tels que représentés sur la figure 1.

Le plateau 22, en l'occurrence en forme de disque, est muni d'au moins deux alvéoles 26 recevant chacun un échantillonneur 24, en l'occurrence un douzaine d'alvéoles, pour réaliser une campagne significative de fixation des différents cations. Ces alvéoles 26 sont répartis suivant au moins un cercle de répartition C.

Lorsque les mesures doivent être réalisées en double ou triple, il est possible de prévoir autant de cercles concentriques équipés eux-mêmes d'alvéoles alignés radialement.

Ce plateau 22 reçoit les échantillonneurs avec la fenêtre 18 du couvercle 16 orientée vers le bas de façon à éviter toute accumulation gravitaire de matières en suspension MES.

Chaque alvéole 26 porte une ouverture 28 vers le bas de façon à permettre l'exposition de la fenêtre 18 des échantillonneurs.

Un joint 30-1 du type joint torique est disposé dans une rainure 32 périphérique à l'ouverture de chaque alvéole et un joint 30-2 périphérique, également du type torique, est disposé dans l'alvéole de façon à faire l'étanchéité autour de l'échantillonneur disposé dans l'alvéole.

L'étanchéité à l'immersion est obtenue par ces joints.

Dans le mode de réalisation préféré, retenu par l'invention, mais sans que ce choix puisse être considéré comme limitatif, le plateau 22 support d'échantillonneurs est mobile en rotation autour d'un axe central XX'.

En dessous de ce plateau 22 support d'échantillonneurs, le dispositif 20 comprend un plateau occultant 32 fixe, en forme de disque également, couvrant la surface du plateau 22 support d'échantillonneurs en faisant étanchéité avec les joints 30-1 de chaque alvéole.

Ce plateau occultant 32 fixe comprend au moins une découpe 34, venue de fabrication et apte à recevoir un éventuel insert annulaire si l'on souhaite modifier le diamètre d'ouverture.

On considère qu'il n'est prévu qu'une seule découpe pour la description qui suit de ce mode de réalisation et sur les dessins.

Cette découpe 34 est ménagée sur le cercle C de même diamètre que celui de répartition des alvéoles 26 de façon à pouvoir être en correspondance exacte avec l'ouverture 28 de chaque alvéole 26 et donc avec la fenêtre 18 de l'échantillonneur 24 qu'il porte.

Dans le cas de plusieurs cercles concentriques d'alvéoles, C1, C2 pour des mesures en double ou C1, C2, C3 pour des mesures en triple, il convient de prévoir autant de découpes 34 que de cercles, ces découpes étant alignées sur des cercles concentriques de même rayon que ceux qui portent les échantillons et suivant un rayon dans le mode de réalisation avec les deux plateaux support d'échantillonneurs et occultant en forme de disque. Ainsi les découpes sont alignées radialement et donc avec un échantillonneur sur chacun des cercles au même moment de façon simultanée et synchronisée nécessairement.

Des moyens moteur MT, du type moteur électrique, sont interposés entre les deux plateaux de façon à permettre un mouvement relatif de rotation des deux plateaux 22 support d'échantillonneurs et occultant 32 fixe l'un par rapport à l'autre. En l'occurrence, un mouvement de rotation du plateau 22 support d'échantillonneurs est établi par rapport au plateau occultant 32 fixe.

Plus particulièrement, les déplacements relatifs permettent de placer n'importe quel alvéole 26 avec son échantillonneur 24 en regard de ladite au moins une découpe 34. Ce sont les positions représentées sur les figures 4 et 5.

Le dispositif 20 comprend de plus, des moyens de pilotage des moyens moteur MT comportant une source d'énergie 40 pour alimenter lesdits moyens moteur, des moyens de mémorisation et de mise en œuvre 42 d'un programme informatique de pilotage et des moyens pour délivrer de l'énergie auxdits moyens moteur en fonction du programme informatique mémorisé.

Le dispositif 20 comprend des moyens de mesure de paramètres physico-chimiques, en l'occurrence au moins un thermomètre, de façon à enregistrer en permanence la température et à pouvoir la corréler avec les durées d'exposition de chaque échantillonneur 24.

Le fonctionnement du dispositif consiste à charger le plateau 22 support d'échantillonneurs 24 en échantillonneurs 24-1 à 24-12 en l'occurrence.

Le plateau 22 support d'échantillonneurs 24, ainsi muni de ses échantillonneurs, est positionné sur le plateau occultant 32 fixe. Un axe central du plateau occultant fixe permet un centrage du plateau 22 support d'échantillonneurs 24 qui est mobile. Aux deux extrémités de cet axe, des moyens de liaison, tels que des œillets, sont rapportés afin d'assurer une suspension supérieure à une bouée et une liaison avec un mouillage au fond, ceci de façon connue et symbolisé sur la figure 2.

Les moyens moteur MT, solidaires du plateau occultant 32 fixe, assurent l'entraînement en rotation du plateau 22 support d'échantillonneurs 24 par exemple à l'aide d'un ensemble pignon / couronne et totalement à la portée de l'homme de l'art.

La source d'énergie 40 peut être un accus associé à un panneau photovoltaïque disposé sur un flotteur ou une structure émergée associée au dispositif selon la présente invention, de façon à recharger ledit accus.

Le programme informatique mémorisé positionne chaque échantillonneur 24-1 à 24-12 en regard de la découpe 34 à l'heure programmée et pendant la durée programmée.

Une fois le premier échantillonneur exposé de façon prévue, un deuxième échantillonneur est positionné à son tour en regard de la découpe 34 pendant la durée programmée.

Ainsi de suite, les échantillonneurs sont exposés dans l'ordre et pendant les durées programmées. C'est ainsi qu'un échantillonneur peut être exposé 3 heures pendant la phase nocturne et un autre échantillonneur peut être exposé pendant 3 heures également pendant la phase diurne et ainsi de suite tous les jours pendant 15 jours.

Un autre échantillonneur peut être exposé pendant 3 jours, un autre pendant les 3 jours suivant, ceci aux mêmes heures.

Toute programmation est possible sauf la superposition des plages horaires disponibles devant l'ouverture du plateau fixe occultant.

Avant et après exposition, tous les échantillonneurs passifs sont hermétiquement isolés du milieu ambiant.

On note que la formation du biofilm BF est totalement interrompue lorsque chaque échantillonneur est en veille dans la position occultée, après exposition.

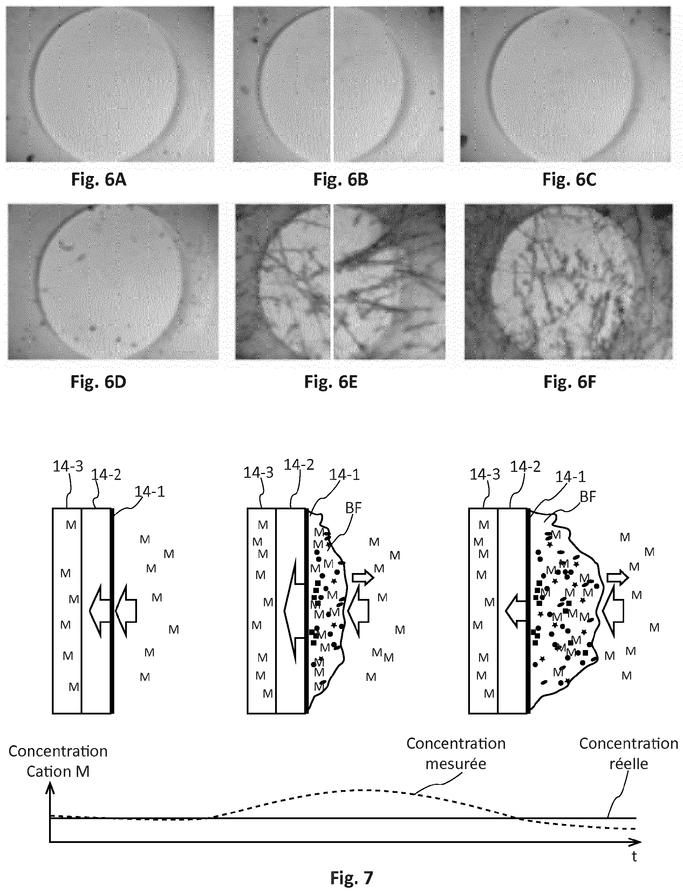

On constate sur les vues 6A à 6F de la figure 6 que le biofilm BF peut se développer rapidement en certains lieux et le développement d'un biofilm provoque une réduction de la surface efficace et modifie la migration des cations métalliques.

Ainsi que cela est représenté schématiquement sur la figure 7, on constate qu'un biofilm BF se développe et colonise rapidement la surface.

Ce biofilm présente d'autres effets plus complexes car non linéaires, voir la courbe représentative.

On a ainsi pu vérifier par des tests sur l'application aux cations métalliques que les cations métalliques CM migrent de façon normale en l'absence d'un biofilm significatif.

Par contre, dès que le biofilm s'épaissit, on constate que les cations métalliques CM sont captés et concentrés par le biofilm, les cations métalliques passent à travers le filtre puis le diffuseur pour être captés par la résine. Il y a une concentration supérieure à la normale, le biofilm jouant un rôle concentrateur qui fausse le résultat en modifiant le taux de transfert à l'excès.

A l'inverse, dès que le biofilm épaissit trop, les cations métalliques CM n'atteignent plus le filtre et donc ultérieurement la résine chimique de capture et ces cations se trouvent repoussés, conduisant à fausser de nouveau le résultat en modifiant le taux de transfert, mais en défaut.

Le procédé de la présente invention consiste à étalonner la formation d'un biofilm en fonction de certains paramètres comme le positionnement géographique, la turbidité, la charge en matière organique, la température, la saison, la luminosité, le pH, la salinité par exemple.

Par ailleurs, le procédé consiste à limiter la durée d'exposition de chaque échantillonneur passif, notamment du type DGT, à la valeur étalonnée durant laquelle la formation du biofilm BF évite toute interaction avec ledit échantillonneur.

De façon avantageuse, un des alvéoles est exempt d'échantillonneur de façon à permettre une occultation complète de tous les échantillonneurs, par exemple s'il est impossible de collecter le dispositif au jour J, pour cause d'intempéries ou d'indisponibilité des moyens logistiques. Les contenus de chaque échantillonneur sont figés, il en est de même entre deux expositions lors de séquences d'expositions successives.

Les dispositif et procédé permettent de répondre à la problématique posée, à savoir la qualité et la reproductibilité des mesures, en plus de tous les avantages pratiques de mise en œuvre. Les dispositif et procédé ont été décrits pour des cations métalliques mais l'application pourrait être diversifiée à des captures d'éléments biochimiques par exemple.

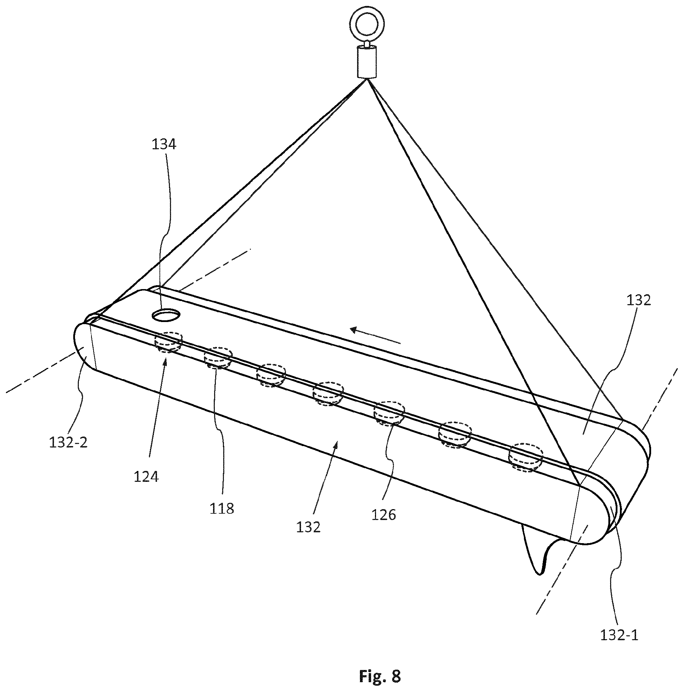

Le dispositif présenté l'a été avec un montage du type barillet avec des plateaux rotatifs en forme de disques mais un dispositif en translation, à crémaillère peut aussi être envisagé de la même façon, cette variante étant représentée sur la figure 8. Les éléments identiques avec le mode de réalisation précédent porte les mêmes références auxquelles on a ajouté 100.

Sur cette figure, on a représenté une succession d'alvéoles 126 recevant des échantillonneurs 124.

Un plateau occultant 132, sous forme d'une bande, est monté en boucle fermée et ladite bande est mise en rotation entre deux poulies 132-1 et 132-2, dont l'une est motrice. La bande peut ainsi tourner entre les deux poulies. Ladite bande est munie d'au moins une découpe 134 de façon à venir devant les fenêtres 118 de chacun des échantillonneurs 124 monté sur une plateau fixe 132 autour duquel tourné la bande du plateau occultant.

La rotation de la bande du plateau occultant permet une exposition desdits échantillonneurs chacun leur tour

On note que dans le mode de réalisation principal à barillet ou de sa variante à translation, il est possible de travailler avec des essais multiples simultanés pour bénéficier de résultats en double ou en triple.

Il suffit alors de ménager trois découpes alignées transversalement.

Ainsi, il est possible d'exposer simultanément trois échantillonneurs dans les mêmes conditions exactement que dans le mode de réalisation principal.

Les couples alvéoles/échantillonneurs sont placés transversalement et alignés. Les découpes viennent alors au droit des rangées transversales d'échantillonneurs.

Afin de maintenir une orientation constante, il est possible d'adjoindre une dérive mécanique tant sur le mode à rotation qu'à translation afin de conserver toujours la même orientation par rapport au courant. Cette dérive mécanique doit être solidaire du plateau occultant à l'opposé de la découpe de façon à maintenir la ou les découpes face au courant et en tête, par exemple.

Pour l'agencement en translation, la dérive est à l'arrière dans le sens longitudinal afin que les trois échantillons soient placés avec la même orientation par rapport au courant.

Le dispositif et le procédé selon la présente invention permet de résoudre les problèmes énoncés en préambule, sans que la fixation d'espèces chimiques soit perturbée par la formation d'un biofilm, avec des mesures sur des plages de temps parfaitement déterminées, avec la possibilité de réexpositions, en évitant la saturation, en facilitant le logistique , en permettant aux opérateurs de s'affranchir des conditions météorologiques, en obtenant une répétitivité certaine, en réalisant des mesures en double ou en triple comme il se doit en contrôle.

## Revendications

1. Dispositif (20) de fixation d'éléments chimiques, notamment des cations métalliques CM en solution dans un milieu aquatique naturel, notamment un milieu marin, le dispositif étant apte à être immergé en milieu naturel aquatique et comportant des échantillonneurs (24, 124), chaque échantillonneur (24) comprenant un boîtier (10), avec une succession de composants incluant une membrane formant filtre (14-1), un gel de diffusion (14-2), et une résine chimique de capture (14-3), apte à capter lesdits éléments chimiques, ainsi qu'une fenêtre (18) d'exposition desdites composants, et un plateau (22, 122) support d'échantillonneurs (24, 124) muni d'au moins deux alvéoles recevant chacun un des échantillonneurs, les échantillonneurs étant reçus dans les alvéoles (26, 126), avec la fenêtre (18) orientée vers le bas,
**caractérisé en ce que** le dispositif comprend également un plateau occultant (32, 132) couvrant la surface du plateau (22, 122) support d'échantillonneurs (24, 124), et muni d'au moins une découpe (34, 134), des moyens moteur MT interposés entre les deux plateaux support d'échantillonneurs et occultant pour engendrer un mouvement relatif d'un plateau par rapport à l'autre, chaque au moins un échantillonneur (24, 124) pouvant venir au droit de ladite découpe (34, 134), et des joints d'étanchéité à l'immersion.

2. Dispositif (20) de fixation d'éléments chimiques, notamment des cations métalliques CM en solution dans un milieu aquatique naturel, notamment un milieu marin, selon la revendication 1, **caractérisé en ce que** les plateaux (22) support d'échantillonneurs (24) et occultant (32) sont en forme de disque et le mouvement relatif est rotatif.

3. Dispositif (20) de fixation d'éléments chimiques, notamment des cations métalliques CM en solution dans un milieu aquatique naturel, notamment un milieu marin, selon la revendication 1, **caractérisé en ce que** les plateaux (122) support d'échantillonneurs (124) et occultant (132) sont en forme de bande et le mouvement relatif est translatif.

4. Dispositif (20) de fixation d'éléments chimiques, notamment des cations métalliques CM en solution dans un milieu aquatique naturel, notamment un milieu marin, selon la revendication 2 ou 3, **caractérisé en ce que** le plateau (22) support d'échantillonneurs (24) est mobile par rapport au plateau occultant (32) qui est fixe.

5. Dispositif (20) de fixation d'éléments chimiques, notamment des cations métalliques CM en solution dans un milieu aquatique naturel, notamment un milieu marin, selon l'une quelconque des revendications 2 ou 4, lorsqu'elle dépend de la revendication 2, **caractérisé en ce que** les alvéoles (26, 126) et la au moins une découpe (34, 134) sont disposés sur au moins un cercle C de même diamètre.

6. Dispositif (20) de fixation d'éléments chimiques, notamment des cations métalliques CM en solution dans un milieu aquatique naturel, notamment un milieu marin, selon la revendication 5, **caractérisé en ce que** les alvéoles (26, 126) et deux découpes (34, 134) sont disposées sur au moins deux cercle C1, C2 de diamètres concentriques.

7. Dispositif (20) de fixation d'éléments chimiques, notamment des cations métalliques CM en solution dans un milieu aquatique naturel, notamment un milieu marin, selon l'une quelconque des revendications précédentes, **caractérisé en ce qu'**il comprend des moyens (38) de pilotage des moyens moteur MT comportant une source d'énergie (40), des moyens de mémorisation et de mise en œuvre (42) d'un programme informatique de pilotage et des moyens pour délivrer de l'énergie auxdits moyens moteur en fonction du programme informatique mémorisé.

8. Dispositif (20) de fixation d'éléments chimiques, notamment des cations métalliques CM en solution dans un milieu aquatique naturel, notamment un milieu marin, selon l'une quelconque des revendications précédentes, **caractérisé en ce qu'**il comprend des moyens de mesure de paramètres physico-chimiques.

9. Dispositif (20) de fixation d'éléments chimiques, notamment des cations métalliques CM en solution dans un milieu aquatique naturel, notamment un milieu marin, selon la revendication 8, **caractérisé en ce qu'**un paramètre physicochimique est la température.

10. Dispositif (20) de fixation d'éléments chimiques, notamment des cations métalliques CM en solution dans un milieu aquatique naturel, notamment un milieu marin, selon l'une quelconque des revendications précédentes, **caractérisé en ce qu'**il comprend une dérive mécanique de façon à conserver une orientation constante par rapport au courant.

11. Procédé de mise en œuvre du dispositif (20) de fixation d'éléments chimiques, notamment des cations métalliques CM en solution dans un milieu aquatique naturel, notamment un milieu marin, selon l'une quelconque des revendications précédentes, **caractérisé en ce qu'**il consiste à étalonner la formation d'un biofilm en fonction de paramètres physico chimiques, géographique et à limiter la durée d'exposition de chaque échantillonneur à la valeur étalonnée durant laquelle la formation du biofilm BF évite toute interaction avec ledit échantillonneur et/ou durant laquelle la résine chimique de capture desdits éléments chimiques reste non saturée.

## Patentansprüche

1. Vorrichtung (20) zur Fixierung chemischer Elemente, insbesondere von metallischen Kationen CM, in Lösung in einem natürlichen aquatischen Medium, insbesondere einem marinen Medium, wobei die Vorrichtung in der Lage ist, in ein natürliches aquatisches Medium eingetaucht zu werden, und Probennehmer (24, 124) umfasst, wobei jeder Probenehmer (24) ein Gehäuse (10) mit einer Abfolge von Komponenten umfasst, einschließlich einer Membran, die einen Filter (14-1) bildet, einem Diffusionsgel (14-2), und einem chemischen Einfangharz (14-3), das in der Lage ist, die chemischen Elemente einzufangen, sowie ein Fenster (18) zur Exposition der Komponenten und eine Platte (22, 122) zur Halterung der Probennehmer (24, 124), die mit mindestens zwei Kammern versehen ist, von denen jede einen der Probennehmer aufnimmt, wobei die Probennehmer in den Kammern (26, 126) mit dem Fenster (18) nach unten ausgerichtet aufgenommen werden,
**dadurch gekennzeichnet, dass** die Vorrichtung außerdem eine Verdeckungsplatte (32, 132) umfasst, die die Oberfläche der Platte (22, 122) zur Halterung von Probennehmern (24, 124) bedeckt und mit mindestens einem Ausschnitt (34, 134) versehen ist, Antriebsmittel MT, die zwischen den beiden Platten zur Halterung von Probennehmern und zur Verdeckung angeordnet sind, um eine Relativbewegung einer Platte in Bezug auf die andere zu bewirken, wobei jeder mindestens eine Probennehmer (24, 124) direkt vor dem Ausschnitt (34, 134) positioniert werden kann, und Immersionsabdichtungen.

2. Vorrichtung (20) zur Fixierung chemischer Elemente, insbesondere von metallischen Kationen CM, in Lösung in einem natürlichen aquatischen Medium, insbesondere einem marinen Medium, nach Anspruch 1,
**dadurch gekennzeichnet, dass** die Platten (22) zur Halterung der Probennehmer (24) und zur Verdeckung (32) scheibenförmig sind und die Relativbewegung rotierend ist.

3. Vorrichtung (20) zur Fixierung chemischer Elemente, insbesondere von metallischen Kationen CM, in Lösung in einem natürlichen aquatischen Medium, insbesondere einem marinen Medium, nach Anspruch 1,
**dadurch gekennzeichnet, dass** die Platten (122) zur Halterung der Probennehmer (124) und zur Verdeckung (132) bandförmig sind und die Relativbewegung translatorisch ist.

4. Vorrichtung (20) zur Fixierung chemischer Elemente, insbesondere von metallischen Kationen CM, in Lösung in einem natürlichen aquatischen Medium, insbesondere einem marinen Medium, nach Anspruch 2 oder 3,
**dadurch gekennzeichnet, dass** die Platte (22) zur Halterung der Probennehmer (24) in Bezug auf die Platte (32) zur Verdeckung, die feststehend ist, beweglich ist.

5. Vorrichtung (20) zur Fixierung chemischer Elemente, insbesondere von metallischen Kationen CM, in Lösung in einem natürlichen aquatischen Medium, insbesondere einem marinen Medium, nach einem der Ansprüche 2 oder 4, wenn sie von Anspruch 2 abhängt, **dadurch gekennzeichnet, dass** die Kammern (26, 126) und der mindestens eine Ausschnitt (34, 134) auf mindestens einem Kreis C mit gleichem Durchmesser angeordnet sind.

6. Vorrichtung (20) zur Fixierung chemischer Elemente, insbesondere von metallischen Kationen CM, in Lösung in einem natürlichen aquatischen Medium, insbesondere einem marinen Medium, nach Anspruch 5,
**dadurch gekennzeichnet, dass** die Kammern (26, 126) und zwei Ausschnitte (34, 134) auf mindestens zwei Kreisen C1, C2 mit konzentrischen Durchmessern angeordnet sind.

7. Vorrichtung (20) zur Fixierung chemischer Elemente, insbesondere von metallischen Kationen CM, in Lösung in einem natürlichen aquatischen Medium, insbesondere einem marinen Medium, nach einem der vorstehenden Ansprüche, **dadurch gekennzeichnet, dass** sie Mittel (38) zur Steuerung der Antriebsmittel MT umfasst, die eine Energiequelle (40), Mittel zum Speichern und Ausführen (42) eines Steuerungscomputerprogramms und Mittel zur Energieversorgung der Antriebsmittel in Abhängigkeit von dem gespeicherten Computerprogramm umfassen.

8. Vorrichtung (20) zur Fixierung chemischer Elemente, insbesondere von metallischen Kationen CM, in Lösung in einem natürlichen aquatischen Medium, insbesondere einem marinen Medium, nach einem der vorstehenden Ansprüche, **dadurch gekennzeichnet, dass** sie Mittel zur Messung physikalisch-chemischer Parameter umfasst.

9. Vorrichtung (20) zur Fixierung chemischer Elemente, insbesondere von metallischen Kationen CM, in Lösung in einem natürlichen aquatischen Medium, insbesondere einem marinen Medium, nach Anspruch 8,
**dadurch gekennzeichnet, dass** ein physikalisch-chemischer Parameter die Temperatur ist.

10. Vorrichtung (20) zur Fixierung chemischer Elemente, insbesondere von metallischen Kationen CM, in Lösung in einem natürlichen aquatischen Medium, insbesondere einem marinen Medium, nach einem der vorstehenden Ansprüche, **dadurch gekennzeichnet, dass** sie eine mechanische Drift umfasst, so dass eine konstante Ausrichtung in Bezug auf die Strömung aufrechterhalten wird.

11. Verfahren zur Implementierung der Vorrichtung (20) zur Fixierung chemischer Elemente, insbesondere von metallischen Kationen CM, in Lösung in einem natürlichen aquatischen Medium, insbesondere einem marinen Medium, nach einem der vorstehenden Ansprüche, **dadurch gekennzeichnet, dass** es darin besteht, die Bildung eines Biofilms in Abhängigkeit von physikalisch-chemischen Parametern, geografisch und die Expositionsdauer jedes Probennehmers auf den kalibrierten Wert zu kalibrieren, währenddessen die Bildung des Biofilms BF jede Wechselwirkung mit dem Probennehmer verhindert und/oder währenddessen das chemische Harz zum Einfangen der chemischen Elemente ungesättigt bleibt.

## Claims

1. Device (20) for fixing chemical elements, in particular metal cations CM, in solution in a natural aquatic environment, in particular a marine environment, the device being suitable for immersion in a natural aquatic environment and comprising samplers (24, 124), each sampler (24) comprising a housing (10), with a succession of components including a filter membrane (14-1), a diffusion gel (14-2), and a chemical capture resin (14-3), capable of capturing said chemical elements, as well as a window (18) for exposure of said components, and a plate (22, 122) supporting samplers (24, 124) provided with at least two cells each receiving one of the samplers, the samplers being received in the cells (26, 126), with the window (18) facing downward,
**characterized in that** the device also comprises a concealing plate (32, 132) covering the surface of the plate (22, 122) supporting samplers (24, 124), and provided with at least one cutout (34, 134), motor means MT interposed between the two sampler-supporting and concealing plates to generate a relative movement of one plate with respect to the other, each at least one sampler (24, 124) being able to come in line with said cutout (34, 134), and immersion seals.

2. Device (20) for fixing chemical elements, in particular metal cations CM, in solution in a natural aquatic environment, in particular a marine environment, according to claim 1, **characterized in that** the plate (22) supporting samplers (24) and concealing plate (32) are disc-shaped and the relative movement is rotary.

3. Device (20) for fixing chemical elements, in particular metal cations CM, in solution in a natural aquatic environment, in particular a marine environment, according to claim 1, **characterized in that** the plate (122) supporting samplers (124) and concealing plate (132) are strip-shaped and the relative movement is translational.

4. Device (20) for fixing chemical elements, in particular metal cations CM, in solution in a natural aquatic environment, in particular a marine environment, according to claim 2 or 3, **characterized in that** the plate (22) supporting samplers (24) is movable relative to the concealing plate (32) which is fixed.

5. Device (20) for fixing chemical elements, in particular metal cations CM, in solution in a natural aquatic environment, in particular a marine environment, according to any of claims 2 or 4, when dependent on claim 2, **characterized in that** the cells (26, 126) and the at least one cutout (34, 134) are arranged on at least one circle C of the same diameter.

6. Device (20) for fixing chemical elements, in particular metal cations CM, in solution in a natural aquatic environment, in particular a marine environment, according to claim 5, **characterized in that** the cells (26, 126) and two cutouts (34, 134) are arranged on at least two circles C1, C2 of concentric diameters.

7. Device (20) for fixing chemical elements, in particular metal cations CM, in solution in a natural aquatic environment, in particular a marine environment, according to any of the preceding claims, **characterized in that** it comprises means (38) for controlling the motor means MT, including an energy source (40), means (42) for storing and implementing a control computer program, and means for delivering energy to said motor means based on the stored computer program.

8. Device (20) for fixing chemical elements, in particular metal cations CM, in solution in a natural aquatic environment, in particular a marine environment, according to any of the preceding claims, **characterized in that** it comprises means for measuring physicochemical parameters.

9. Device (20) for fixing chemical elements, in particular metal cations CM, in solution in a natural aquatic environment, in particular a marine environment, according to claim 8, **characterized in that** a physicochemical parameter is temperature.

10. Device (20) for fixing chemical elements, in particular metal cations CM, in solution in a natural aquatic environment, in particular a marine environment, according to any of the preceding claims, **characterized in that** it comprises a mechanical fin so as to maintain a constant orientation with respect to the current.

11. Method for implementing the device (20) for fixing chemical elements, in particular metal cations CM, in solution in a natural aquatic environment, in particular a marine environment, according to any of the preceding claims, **characterized in that** it consists in calibrating the formation of a biofilm based on physicochemical, geographical parameters and in limiting the duration of exposure of each sampler to the calibrated value during which the formation of the biofilm BF avoids any interaction with said sampler and/or during which the chemical resin for capturing said chemical elements remains unsaturated.
